# EUROPÄISCHE PATENTSCHRIFT

(11) **EP 0 728 753 B1**
(45) Veröffentlichungstag und Bekanntmachung des Hinweises auf die Patenterteilung: **16.09.1998**
(21) Anmeldenummer: 96102308.2
(22) Anmeldetag: 16.02.1996
(51) Int. Cl.: C07D 309/30, A01N 43/16

(54) **5-Tetrahydropyranon-Cyclohexenonoximether und deren Verwendung als Herbizide**
5-Tetrahydropyranone-cyclohexenone oxime ethers and their use as herbicides
Ethers de 5-tétrahydropyranone-cyclohexénonoxim et leurs utilisation comme herbicides

(30) Priorität: 24.02.1995 DE 19506570
(43) Veröffentlichungstag der Anmeldung: 28.08.1996
(73) Patentinhaber: BASF AKTIENGESELLSCHAFT, 67056 Ludwigshafen (DE)
(72) Erfinder: Rheinheimer, Joachim, Dr., D-67063 Ludwigshafen (DE); Misslitz, Ulf, Dr., D-67433 Neustadt (DE); Kardorff, Uwe, Dr., D-68159 Mannheim (DE); Westphalen, Karl-Otto, Dr., D-67346 Speyer (DE); Walter, Helmut, Dr., D-67283 Obrigheim (DE)

(56) Entgegenhaltungen:
- EP-A- 0 142 741
- WO-A-93/16033

## Beschreibung

Die vorliegende Erfindung betrifft neue 5-Tetrahydropyranon-Cyclohexenonoximether der Formel I 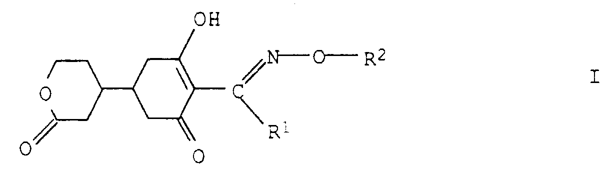 in der die Substituenten folgende Bedeutung haben:
- R¹: eine C₁-C₆-Alkylgruppe;
- R²: die Phenylgruppe, die unsubstituiert sein oder ein bis drei Substituenten tragen kann, jeweils ausgewählt aus der Gruppe bestehend aus Nitro, Cyano, Halogen, C₁-C₄-Alkyl und C₁-C₄-Halogenalkyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
Halogen, C₁-C₃-Alkyl,
Phenyl, das gewünschtenfalls seinerseits ein bis drei Reste tragen kann, jeweils ausgewählt aus der Gruppe bestehend aus Nitro, Cyano, Halogen, C₁-C₄-Alkyl, C₁-C₄-Halogenalkyl, Phenyl und Phenoxy, oder
Phenoxy, das gewünschtenfalls seinerseits ein bis drei Reste tragen kann, jeweils ausgewählt aus der Gruppe bestehend aus Nitro, Cyano, Halogen, C₁-C₄-Alkyl und C₁-C₄-Halogenalkyl;
sowie die landwirtschaftlich brauchbaren Salze von I und die Ester von I mit C₁-C₁₀-Carbonsäuren oder anorganischen Säuren.

Außerdem betrifft die Erfindung die Verwendung dieser Verbindungen als Herbizide, herbizide Mittel, welche diese Verbindungen als wirksame Substanzen enthalten, Verfahren zur Herstellung dieser herbiziden Mittel sowie Verfahren zur Bekämpfung von unerwünschtem Pflanzenwuchs mit den Verbindungen I.

Aus der Literatur sind bereits herbizid wirksame Cyclohexandione der Formel I' 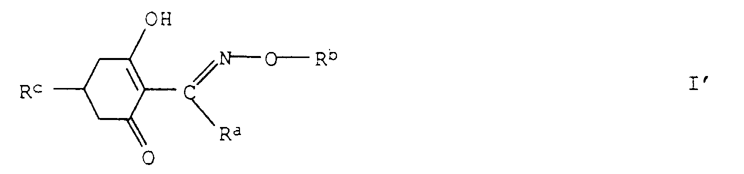 bekannt, wobei R^{a}, R^{b} und R^{c} u. a. für die folgenden Bedeutungen stehen:
- EP-A 142 741 (R^{a} = C₁-C₄-Alkyl; R^{b} = C₁-C₄-Alkyl, C₃-/C₄-Alkenyl, das 1-3 Halogenatome tragen kann, Propargyl; R^{c} = Tetrahydropyran-4-yl);
- DE-A 38 38 309 (R^{a} = C₁-C₆-Alkyl; R^{b} = substituierter 4-Phenylbutylen- oder 4-Phenylbutenylenrest; R^{c} = substituierter 5- bis 7-gliedriger Heterocyclus);
- EP-A 456 112 (R^{a} = C₁-C₆-Alkyl; R^{b} = substituierter 3-Phenoxypropylen- oder 2-Phenoxyethylenrest; R^{c} = substituierter 5- bis 7-gliedriger Heterocyclus).

Da die herbiziden Eigenschaften der bekannten Verbindungen, insbesondere hinsichtlich ihrer Selektivität gegen Ungräser in grasartigen Kulturpflanzen, nicht immer voll befriedigen, lagen der Erfindung neue Cyclohexenonoximether zugrunde, mit denen sich Ungräser in grasartigen Kulturen wie Reis und Mais besser als bisher gezielt bekämpfen lassen.

Demgemäß wurden die eingangs definierten 5-Tetrahydropyranon-Cyclohexenonoximether I gefunden. Außerdem wurden ihre Verwendung als Herbizide, herbizide Mittel, die die Verbindungen I enthalten, ein Verfahren zur Herstellung dieser Mittel sowie ein Verfahren zur Bekämpfung von unerwünschtem Pflanzenwuchs mit den Verbindungen I gefunden.

Die 5-Tetrahydropyranon-Cyclohexenonoximether I sind auf verschiedene Weise erhältlich, und zwar bevorzugt in an sich bekannter Weise aus schon bekannten Cyclohexenonen der Formel II gemäß folgendem Reaktionsschema:

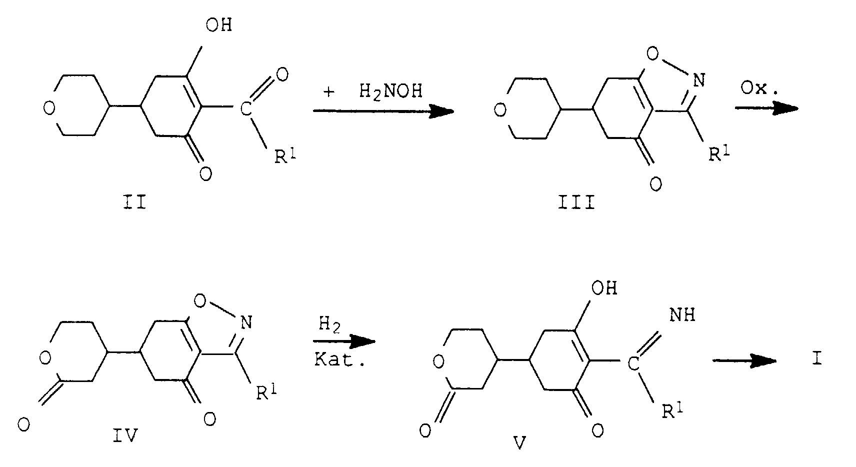

Vorzugsweise setzt man II mit einem geeigneten Salz des Hydroxylamins, insbesondere dessen Hydrochlorid, um. Als besonders zweckmäßiges Lösungsmittel hat sich hierbei Wasser erwiesen.

Die Reaktionsführung erfolgt in Gegenwart einer Base, wobei normalerweise eine Basenmenge von etwa 0,5 bis 2 Mol-Äquivalent, bezogen auf die Ammoniumverbindung, ausreichend ist.

Als Basen kommen z.B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid oder Calciumoxid in Betracht. Des weiteren sind organische Basen wie Pyridin und tert.-Amine wie Triethylamin geeignet.

Eine Variante des Verfahrens besteht darin, II ohne Base mit der freien Hydroxylaminbase H₂NOH, z.B. in Form einer wäßrigen Lösung, umzusetzen; je nach verwendetem Lösungsmittel erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril und cyclische Ether wie Dioxan und Tetrahydrofuran.

Zweckmäßigerweise setzt man das Cyclohexenon II und das Hydroxylamin bzw. dessen Salz in etwa stöchiometrischen Mengen ein, jedoch kann auch ein Überschuß der einen oder anderen Komponente, bis ca. 100 mol-%, vorteilhaft sein.

Die Reaktionstemperatur liegt im allgemeinen zwischen 0°C und dem Siedepunkt des Reaktionsgemisches, vorzugsweise bei 20 bis 80°C.

Als Oxidationsmittel für die Oxidation von III zum Lacton IV ist z.B. Chromtrioxid geeignet. Normalerweise gelingt die Oxidation bei Temperaturen von 0^{o}C bis zur Siedetemperatur des Reaktionsgemisches.

Als Lösungsmittel ist Acetanhydrid besonders geeignet, jedoch sind z.B. auch halogenierte Kohlenwasserstoffe wie Methylenchlorid brauchbar.

Das Oxidationsmittel wird normalerweise in mindestens äquimolaren Mengen, bezogen auf III, eingesetzt. Im allgemeinen hat sich aber ein großer Überschuß an Oxidationsmittel als besonders vorteilhaft erwiesen.

Die Hydrierung von IV wird normalerweise in einem für derartige Reaktionen üblichen Lösungs- oder Verdünnungsmittel vorgenommen, beispielsweise in einem Alkohol wie Methanol, Ethanol, n-Propanol und Isopropanol, in einem Ether wie Diethylether und Tetrahydrofuran oder in einem Ester wie Ethylacetat. Auch Gemische dieser Lösungsmittel kommen in Betracht.

Als Hydrierungsmittel kommt insbesondere molekularer Wasserstoff in Gegenwart eines geeigneten Edelmetallkatalysators wie Palladium (auf Kohle) oder Platin in Betracht.

In der Regel verwendet man den Wasserstoff im Überschuß und arbeitet bei einem Druck von etwa 1 bis 200 bar, vorzugsweise von 1 bis 50 bar.

Die Reaktionstemperatur liegt allgemein bei (-78) bis etwa 130°C, bevorzugt bei 0 bis 100°C.

Die Überführung von V in I kann in Analogie zu ähnlichen, in EP-A 368 227, EP-A 456 112, U.S. 4,249,937 und WO 92/08696 beschriebenen Umsetzungen erfolgen.

Sofern nicht anders angegeben, werden die vorstehend beschriebenen Reaktionsschritte zweckmäßigerweise bei Normaldruck oder unter dem Eigendruck des jeweiligen Verdünnungsmittels vorgenommen.

Die erfindungsgemäßen 5-Tetrahydropyranon-Cyclohexenonoximether I können in Form ihrer landwirtschaftlich brauchbaren Salze oder als Enolester vorliegen, wobei es auf die Art des Salzes oder Esters im allgemeinen nicht ankommt. In der Regel sind zur Salzbildung solche Basen und zur Veresterung solche Säuren geeignet, welche die herbizide Wirkung von I nicht negativ beeinträchtigen.

Alkalimetallsalze der Verbindungen I können durch Behandeln der 3-Hydroxycyclohexenon-Verbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphonium-, Sulfonium- und Sulfoxoniumsalze mittels Ammonium-, Phosphonium-, Sulfonium- oder Sulfoxoniumhydroxiden.

Die Ester der Verbindungen I sind ebenfalls in üblicher Weise erhältlich (vgl. z.B. Organikum, VEB Deutscher Verlag der Wissenschaften, 17. Auflage, Berlin 1988, S. 405 - 408).

In Abhängigkeit von der Bedeutung von R² und der Stellung des Pyranon-Rings am Cyclohexanon-Grundkörper können die 5-Tetrahydropyranon-Cyclohexenonoximether I bei der Herstellung als Isomerengemisch anfallen, wobei sowohl E-/Z-Isomerengemische als auch (R-/S-)Enantiomeren- oder Diastereoisomerengemische möglich sind. Die Isomerengemische können gewünschtenfalls nach den hierfür üblichen Methoden, z.B. durch Chromatographie oder durch Kristallisation, getrennt werden.

Die 5-Tetrahydropyranon-Cyclohexenonoximether I können in mehreren tautomeren Formen geschrieben werden, die alle von der Erfindung umfaßt werden:

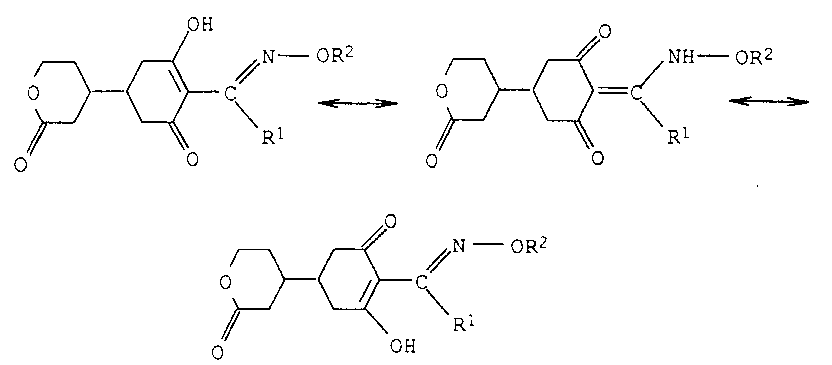

Die in den Definitionen der Substituenten verwendeten Sammelbegriffe
Halogen, Alkyl, Halogenalkyl, Alkenyl und Alkinyl
stellen Kurzschreibweisen für eine individuelle Aufzählung der einzelnen Gruppenmitglieder dar. Sämtliche Alkyl-, Halogenalkyl-, Alkenyl- und Alkinylteile können geradkettig oder verzweigt sein. Die Halogenalkylteile können gleiche oder verschiedene Halogenatome tragen.

Im einzelnen bedeuten beispielsweise
- Halogen: Fluor, Chlor, Brom, Jod;
- C₁-C₄-Alkyl(gruppe) : Methyl, Ethyl, n-Propyl, 1-Methylethyl, n-Butyl, 1-Methylpropyl, 2-Methylpropyl, 1,1-Dimethylethyl;
- C₁-C₃-Alkyl: Methyl, Ethyl, n-Propyl, 1-Methylethyl;
- C₁-C₄-Halogenalkyl: C₁-C₄-Alkyl wie vorstehend genannt, das partiell oder vollständig durch Fluor, Chlor und/oder Brom substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonafluorbutyl;
- C₃-C₄-Alkenylgruppe: 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl;
- C₃-C₄-Alkinylgruppe: Prop-1-in-1-yl, Prop-2-in-3-yl, n-But-1-in-1-yl, n-But-1-in-4-yl, n-But-2-in-1-yl.

Im Hinblick auf die herbizide Wirksamkeit der 5-Tetrahydropyranon-Cyclohexenonoximether I sind die folgenden Bedeutungen der Substituenten, und zwar für sich allein oder in Kombination, besonders bevorzugt:
- R¹: Ethyl und Propyl;
- R²: die Phenylgruppe, unsubstituiert oder ein- bis dreifach substituiert durch
- Nitro, Cyano;
- Halogen, insbesondere Fluor, Chlor;
- C₁-C₄-Alkyl, insbesondere Methyl;
- C₁-C₄-Halogenalkyl, insbesondere Trifluormethyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
- Halogen, insbesondere Fluor, Chlor;
- C₁-C₃-Alkyl, insbesondere Methyl;
- Phenyl, das unsubstituiert sein oder ein bis drei Substituenten tragen kann, ausgewählt aus der Gruppe bestehend aus
   - Nitro, Cyano;
   - Halogen, insbesondere Fluor, Chlor;
   - C₁-C₄-Alkyl, insbesondere Methyl;
   - C₁-C₄-Halogenalkyl, insbesondere Trifluormethyl;
   - Phenyl, Phenoxy;
- Phenoxy, das unsubstituiert sein oder ein bis drei Substituenten tragen kann, ausgewählt aus der Gruppe bestehend aus
   - Nitro, Cyano;
   - Halogen, insbesondere Fluor, Chlor;
   - C₁-C₄-Alkyl, insbesondere Methyl;
   - C₁-C₄-Halogenalkyl, insbesondere Trifluormethyl; ganz besonders bevorzugt sind Ethyl, Chlorallyl, 2-Phenoxypropyl, 3-Phenylpropenyl und 4-Phenylbutenyl, wobei die Phenylringe unsubstituiert sein oder ein bis drei Chlor- und/oder Fluoratome tragen können.

Als Salze der 5-Tetrahydropyranon-Cyclohexenonoximether der Formel I kommen landwirtschaftlich brauchbare Salze, beispielsweise Alkalimetallsalze, insbesondere das Natrium- oder Kaliumsalz, Erdalkalimetallsalze, insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Unter landwirtschaftlich brauchbaren Estern sind vorzugsweise die Ester von C₁-C₁₀-Fettsäuren, insbesondere C₁-C₆-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethylcarbonsäure, Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethyl-butylcarbonsäure, l-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methyl-propylcarbonsäure und 1-Ethyl-2-methylpropylcarbonsäure, zu verstehen.

Die 5-Tetrahydropyranon-Cyclohexenonoximether I, deren Salze und Ester, eignen sich - sowohl als Isomerengemische als auch in Form der reinen Isomeren - als Herbizide. Im allgemeinen sind sie verträglich und somit selektiv in breitblättrigen Kulturen sowie in monokotylen (einkeimblättrigen) Gewächsen, welche nicht zu den Gramineen zählen. Einige der erfindungsgemäßen Verbindungen I sind auch zur selektiven Bekämpfung von unerwünschten Gräsern in Gramineenkulturen geeignet. Dieser Effekt tritt vor allem bei niedrigen Aufwandmengen auf.

Darüber hinaus können die Verbindungen I auch in Kulturen, die durch Züchtung einschließlich gentechnischer Methoden gegen die Wirkung von Herbiziden tolerant sind, verwandt werden.

Die 5-Tetrahydropyranon-Cyclohexenonoximether I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, 5 Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen von 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 8 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 10 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. 19 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 26 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 12 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.
VIII. 1 Gewichtsteil der Verbindung Nr. 24 wird in einer Mischung gelöst, die aus 80 Gewichtsteilen Cyclohexanon und 20 Gewichtsteilen Emulphor El¹⁾ besteht. Man erhält ein stabiles Emulsionskonzentrat.

¹⁾ ethoxyliertes Ricinusöl (caster-oil)

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha mindestens einer aktiven Substanz (a.S.) der Formel I.

In Anbetracht der Vielseitigkeit der Applikationsmethoden können die 5-Tetrahydropyranon-Cyclohexenonoximether I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte können die 5-Tetrahydropyranon-Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

### 2-(1-(2-[4-Chlorphenoxy)propoxy]imino-propyl)-3-hydroxy-5-(tetrahydropyran-2-on-4-yl)-2-cyclohexen-1-on (Verbindung Nr. 1)

a) 3-Ethyl-6-(tetrahydropyran-4-yl)-6,7-dihydro-5H-benzo[d]isoxazol-4-on:
   Zu 34,8 g (0,5 Mol) Hydroxylaminhydrochlorid, 41,0 g (0,5 Mol) Natriumacetat und 10 ml 50 gew.-%iger Natronlauge in 500 ml Wasser tropfte man unter Rühren eine Lösung von 113,4 g (0,44 Mol) 3-Hydroxy-2-propionyl-5-(tetrahydropyran-4-yl)-2-cyclohexen-1-on in 1 l Ethanol. Anschließend wurde die Reaktionsmischung auf 80°C erwärmt und bei dieser Temperatur 2 Std. gerührt. Danach ließ man auf ca. 20°C abkühlen. Das Produkt wurde 3 mal mit Methyl-tert.-butylether extrahiert und, nach Trocknen der Etherphase über Natriumsulfat, durch Entfernen des Ethers bei reduziertem Druck isoliert. Die Reinigung des Rohprodukts erfolgte chromatographisch an Kieselgel-60 (Laufmittel: Methyl-tert.-butylether/Ethylacetat). Ausbeute: 65 g.
b) 3-Ethyl-6-(tetrahydropyran-2-on-4-yl)-6,7-dihydro-5H-benzo[d]isoxazol-4-on:
   Zu 30 g (0,12 Mol) 3-Ethyl-6-(tetrahydropyran-4-yl)-6,7-dihydro-5H-benzo[d]isoxazol-4-on in 300 ml Acetanhydrid wurden 12 g (0,12 Mol) Chromtrioxid gegeben, wobei sich die Mischung auf 70°C erwärmte. In Abständen von jeweils 1 Std. wurden noch zweimal je 6 g (0,06 Mol) Chromtrioxid zugegeben, wonach man das Reaktionsgemisch noch 4 Std. bei etwa 20° rührte. Anschließend wurde die Mischung in 1 l Eiswasser eingerührt. Danach extrahierte man das Produkt mit Ethylacetat. Die organische Phase wurde mit Wasser und einer konz. Kochsalzlösung gewaschen, über Natriumsulfat getrocknet und schließlich eingeengt. Die Reinigung des Rohprodukts erfolgte chromatographisch an Kieselgel-60 (Laufmittel: Methyl-tert.-butylether/Ethylacetat). Ausbeute: 22 g.
c) 3-Hydroxy-2-(1-iminopropyl)-5-(tetrahydropyran-2-on-4-yl)-2-cyclohexen-1-on:
   Zu 20 g (0,08 Mol) 3-Ethyl-6-(tetrahydropyran-2-on-4-yl)-6,7-dihydro-5H-benzo[d]isoxazol-4-on, 6,4 g (0,06 Mol) Triethylamin und 1,6 g Palladium auf Aktivkohle (10 %) in 100 ml Ethanol wurde bei 40°C 24 Std. lang Wasserstoff mit einem Druck von 5 gegeben. Anschließend filtrierte man die ungelösten Bestandteile ab, wonach das Filtrat bei reduziertem Druck eingeengt wurde. Die Reinigung des Rückstandes erfolgte chromatographisch an Kieselgel-60 (Laufmittel: Methyl-tert.-butylether/Isopropanol). Ausbeute: 15 g.
d) Wertprodukt:
   Eine Mischung aus 7,0 g (0,02 Mol) 3-Hydroxy-2-(1-iminopropyl)-5-(tetrahydropyran-2-on-4-yl)-2-cyclohexen-1-on, 3,7 g (0,02 Mol) O-(2-(4-Chlorphenoxy)-propyl)hydroxylamin und 100 ml Methanol wurde 5 Std. bei ca. 20°C gerührt, wonach man die Reaktionsmischung bei reduziertem Druck einengte. Die Reinigung des Rohproduktes erfolgte chromatographisch an Kieselgel-60 (Laufmittel: Methyl-tert.-butylether/Ethylacetat). Ausbeute: 2,2 g (Öl, ¹H-NMR siehe Tabelle 1).

In der folgenden Tabelle 1 sind weitere 5-Tetrahydropyranon-Cyclohexenonoximether I aufgeführt, die auf die gleiche Weise hergestellt wurden oder herstellbar sind:

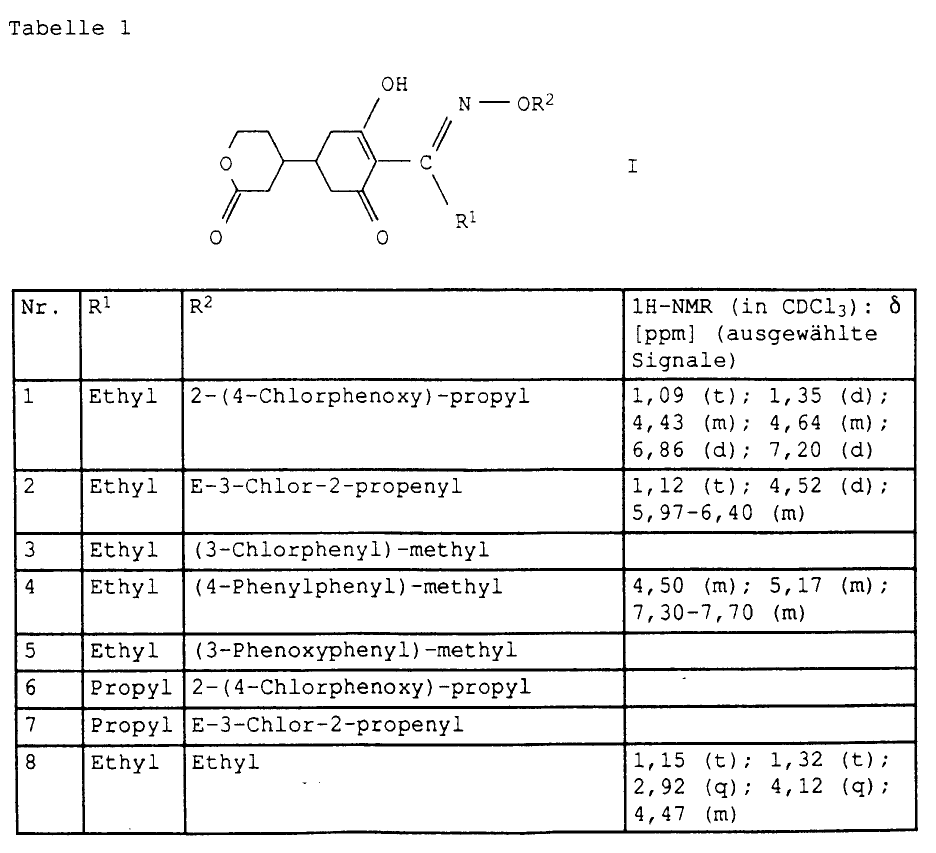

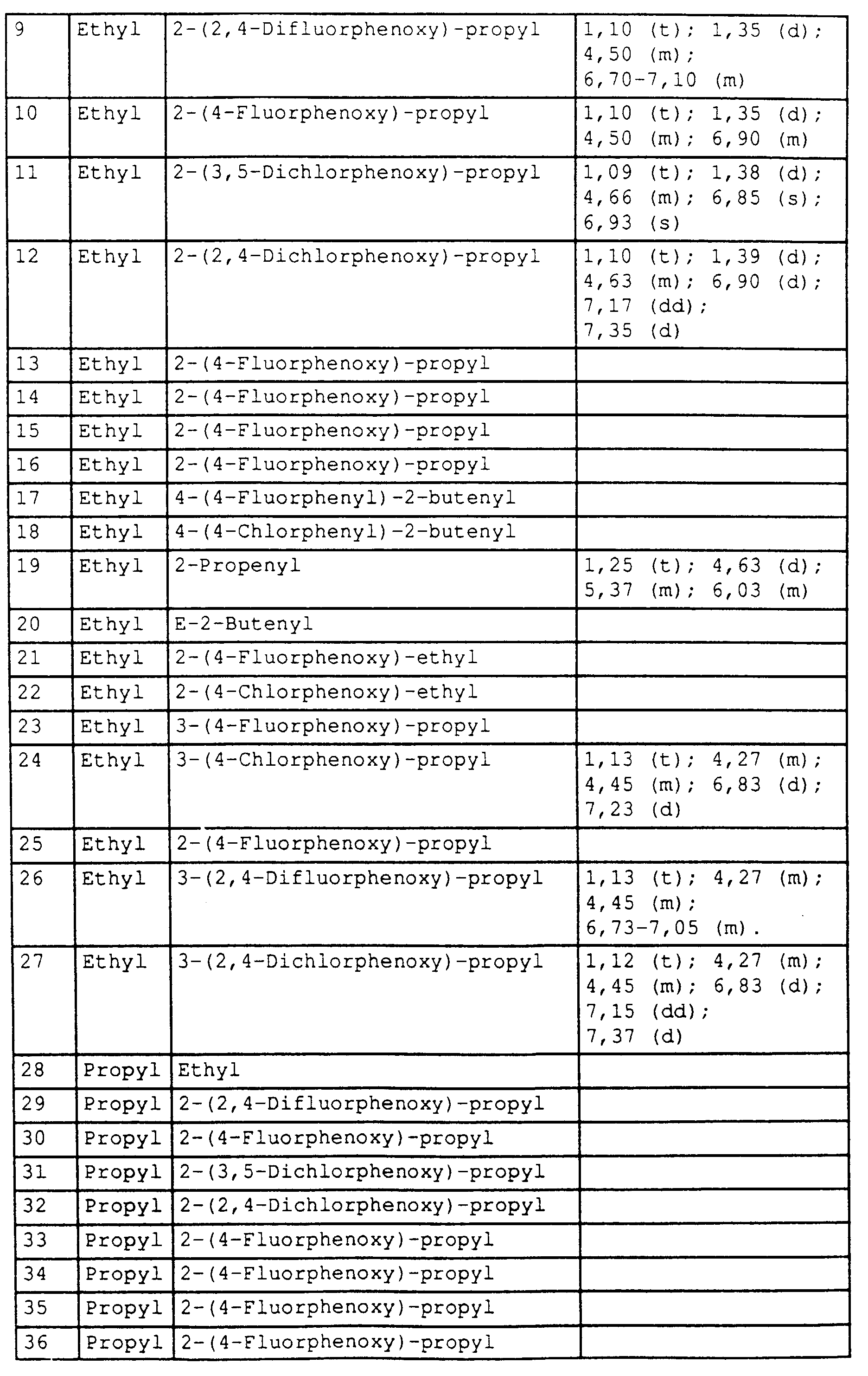

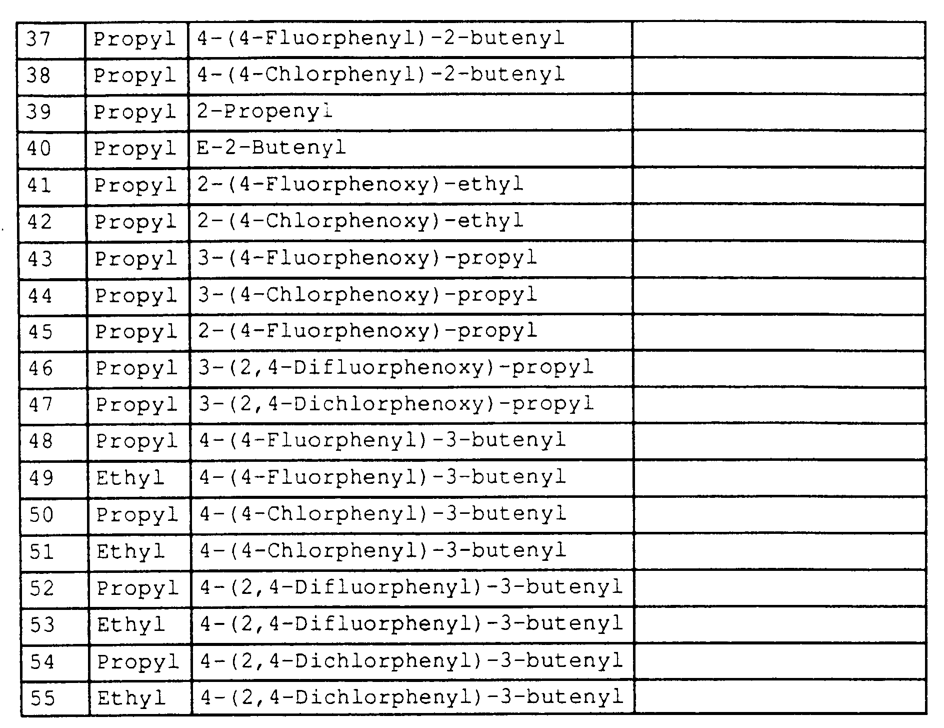

### Anwendungsbeispiele

Die herbizide Wirkung der 5-Tetrahydropyranon-Cyclohexenonoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung werden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Aufwandmenge für die Nachauflaufbehandlung betrug 0,25 und 0,125 kg/ha an aktiver Substanz (a.S.).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Digitaria sanguinalis | Blutfingerhirse | crabgrass |
| Setaria faberii | Borstenhirse | giant foxtail |
| Setaria italica | Kolbenhirse | foxtail millet |
| Setaria viridis | Grüne Borstenhirse | green foxtail |
| Triticum aestivum | Winterweizen | winter wheat |

Das Ergebnis zeigte, daß mit den Verbindungen Nr. 2 und 26 unerwünschte Gräser in Weizen als Beispielkulturen sehr gut bekämpft werden können. Die aus der EP-A 142 741 bekannte Vergleichsverbindung A 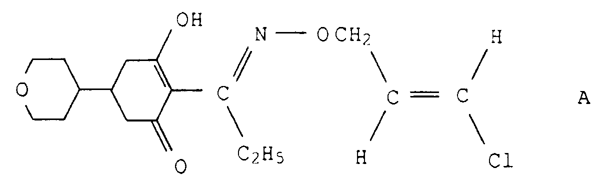 und die aus der WO 93/16033 bekannte Vergleichsverbindung B 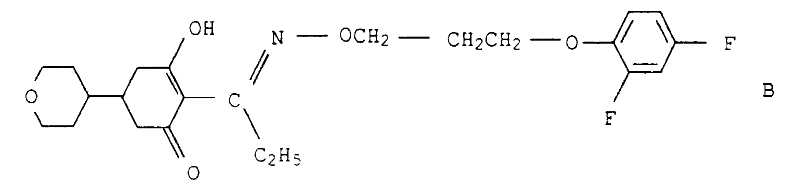 schädigten dagegen sowohl die Ungräser als auch die Kulturpflanze.

## Patentansprüche

1. 5-Tetrahydropyranon-Cyclohexenonoximether der Formel I 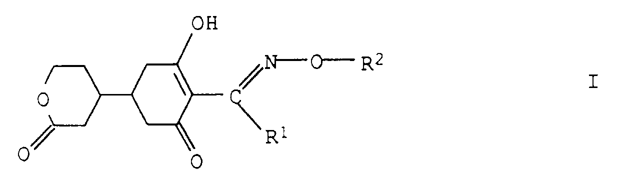 in der die Substituenten folgende Bedeutung haben:
R¹ eine C₁-C₆-Alkylgruppe;
R² die Phenylgruppe, die unsubstituiert sein oder ein bis drei Substituenten tragen kann, jeweils ausgewählt aus der Gruppe bestehend aus Nitro, Cyano, Halogen, C₁-C₄-Alkyl und C₁-C₄-Halogenalkyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
Halogen, C₁-C₃-Alkyl, Phenyl, das gewünschtenfalls seinerseits ein bis drei Reste tragen kann, jeweils ausgewählt aus der Gruppe bestehend aus Nitro, Cyano, Halogen, C₁-C₄-Alkyl, C₁-C₄-Halogenalkyl, Phenyl und Phenoxy, oder
Phenoxy, das gewünschtenfalls seinerseits ein bis drei Reste tragen kann, jeweils ausgewählt aus der Gruppe bestehend aus Nitro, Cyano, Halogen, C₁-C₄-Alkyl und C₁-C₄-Halogenalkyl;
sowie die landwirtschaftlich brauchbaren Salze von I und die Ester von I mit C₁-C₁₀-Carbonsäuren oder anorganischen Säuren.

2. Verwendung der 5-Tetrahydropyranon-Cyclohexenonoximether I und/oder deren landwirtschaftlich brauchbaren Salze und/oder Ester mit C₁-C₁₀-Carbonsäuren oder anorganischen Säuren, gemäß Anspruch 1, als Herbizide.

3. Herbizides Mittel, enthaltend eine herbizid wirksame Menge mindestens eines 5-Tetrahydropyranon-Cyclohexenonoximethers der Formel I und/oder eines landwirtschaftlich brauchbaren Salzes von I und/oder eines Esters von I mit einer Säure, gemäß Anspruch 1, und mindestens einen inerten flüssigen und/oder festen Trägerstoff sowie gewünschtenfalls mindestens einen oberflächenaktiven Stoff.

4. Verfahren zur Herstellung von herbizid wirksamen Mitteln, dadurch gekennzeichnet, daß man eine herbizid wirksame Menge mindestens eines 5-Tetrahydropyranon-Cyclohexenonoximethers der Formel I und/oder eines landwirtschaftlich brauchbaren Salzes von I und/oder eines Esters von I mit einer Säure, gemäß Anspruch 1, mit mindestens einem inerten flüssigen und/oder festen Trägerstoff und gewünschtenfalls mit mindestens einem oberflächenaktiven Stoff mischt.

5. Verfahren zur Bekämpfung von unerwünschtem Pflanzenwuchs, dadurch gekennzeichnet, daß man eine herbizid wirksame Menge mindestens eines 5-Tetrahydropyranon-Cyclohexenonoximethers der Formel I und/oder eines landwirtschaftlich brauchbaren Salzes von I und/oder Esters von I mit einer Säure, gemäß Anspruch 1, auf Pflanzen, deren Lebensraum oder auf Saatgut einwirken läßt.

## Claims

1. A 5-tetrahydropyranone cyclohexenone oxime ether of the formula I 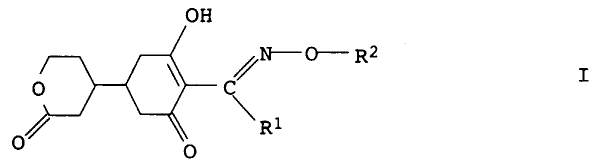 where the substituents have the following meanings:
R¹ is a C₁-C₆-alkyl group;
R² is the phenyl group, which can be unsubstituted or can have attached to it one to three substituents, in each case selected from the group consisting of nitro, cyano, halogen, C₁-C₄-alkyl and C₁-C₄-haloalkyl;
a C₁-C₄-alkyl, C₃-C₄-alkenyl or C₃-C₄-alkynyl group, it being possible, if desired, for these groups to have attached to them one of the following substituents:
halogen, C₁-C₃-alkyl, phenyl which, if desired, can have attached to it, in turn, one to three radicals, in each case selected from the group consisting of nitro, cyano, halogen, C₁-C₄-alkyl, C₁-C₄-haloalkyl, phenyl and phenoxy, or
phenoxy which, if desired, can have attached to it, in turn, one to three radicals, in each case selected from the group consisting of nitro, cyano, halogen, C₁-C₄-alkyl and C₁-C₄-haloalkyl;
or an agriculturally useful salt of I or an ester of I with a C₁-C₁₀-carboxylic acid or inorganic acid.

2. The use of the 5-tetrahydropyranone cyclohexenone oxime ethers I and/or their agriculturally useful salts and/or esters with C₁-C₁₀-carboxylic acids or inorganic acids, as claimed in claim 1, as herbicides.

3. A herbicidal composition comprising a herbicidally active amount of at least one 5-tetrahydropyranone cyclohexenone oxime ether of the formula I and/or of an agriculturally useful salt of I and/or an ester of I with an acid, as claimed in claim 1, and at least one inert liquid and/or solid carrier and, if desired, at least one surfactant.

4. Process for the preparation of herbicidally active compositions, which comprises mixing a herbicidally active amount of at least one 5-tetrahydropyranone cyclohexenone oxime ether of the formula I and/or of an agriculturally useful salt of I and/or an ester of I with an acid, as claimed in claim 1, with at least one inert liquid and/or solid carrier and, if desired, at least one surfactant.

5. A method of controlling undesirable vegetation, which comprises allowing to act a herbicidally active amount of at least one 5-tetrahydropyranone cyclohexenone oxime ether of the formula I and/or of an agriculturally useful salt of I and/or an ester of I with an acid, as claimed in claim 1, on plants, their environment or seed.

## Revendications

1. Ethers de 5-tétrahydropyrannone-cyclohexénonoximes de formule I 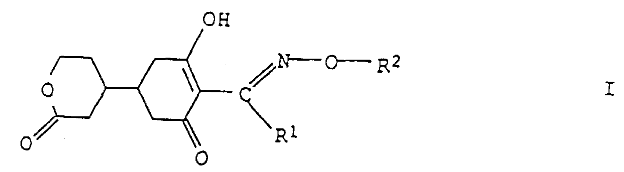 dans laquelle les symboles ont les significations suivantes :
R¹ représente un groupe alkyle en C1-C6;
R² représente un groupe phényle non substitué ou portant un à trois substituants choisis parmi les groupes nitro, cyano, les halogènes, les groupes alkyle en C1-C4 et halogénoalkyle en C1-C4;
un groupe alkyle en C1-C4, alcényle en C3-C4 ou alcynyle en C3-C4, portant éventuellement l'un des substituants suivants :
un halogène, un groupe alkyle en C1-C3, un groupe phényle qui peut lui-même porter un à trois substituants choisis parmi les groupes nitro, cyano, les halogènes, les groupes alkyle en C1-C4, halogénoalkyle en C1-C4, phényle ou phénoxy, ou bien un groupe phénoxy qui peut lui-même porter un à trois substituants choisis parmi les groupes nitro, cyano, les halogènes, les groupes alkyle en C1-C4 et halogénoalkyle en C1-C4 ;
et les sels de I et esters de I et d'acides carboxyliques en C1-C10 ou d'acides minéraux convenant pour les applications agricoles.

2. Utilisation des éthers de 5-tétrahydropyrannone-cyclohexénonoximes I et/ou de leurs sels et/ou esters d'acides carboxyliques en C1-C10 ou d'acides minéraux, convenant pour les applications agricoles, selon la revendication 1, en tant qu'herbicides.

3. Produit herbicide contenant une quantité herbicide efficace d'au moins un éther de 5-tétrahydropyrannone-cyclohexénonoxime de formule I et/ou d'un sel de I et/ou d'un ester de I et d'un acide, convenant pour les applications agricoles, selon la revendication 1, et au moins un véhicule liquide et/ou solide inerte et le cas échéant au moins une substance tensio-active.

4. Procédé pour la préparation des produits herbicides, caractérisé par le fait que l'on mélange une quantité herbicide efficace d'au moins un éther de 5-tétrahydropyrannone-cyclohexénonoxime de formule I et/ou d'un sel de I et/ou d'un ester de I et d'un acide, convenant pour les applications agricoles, selon la revendication 1, avec au moins un véhicule liquide et/ou solide inerte et, si on le désire, au moins une substance tensio-active.

5. Procédé pour combattre les croissances des végétaux indésirables, caractérisé par le fait que l'on fait réagir sur les végétaux, leur habitat ou leurs semences, une quantité herbicide efficace d'au moins un éther de 5-tétrahydro-pyrannone-cyclohexénonoxime de formule I et/ou d'un sel de I et/ou d'un ester de I et d'un acide, convenant pour les applications agricoles, selon la revendication 1.
